# EUROPEAN PATENT SPECIFICATION

(11) **EP 0 697 889 B1**
(45) Date of publication and mention of the grant of the patent: **13.11.2002**
(21) Application number: 94913873.9
(22) Date of filing: 25.01.1994
(51) Int. Cl.: A61K 39/395, C07K 16/28, C12N 5/20, C12P 21/08, C12N 15/02

(54) **COMBINED TREATMENT OF IRON DEPLETION AND IgG ANTIBODY**
BEHANDLUNG MITTELS EISEN-ENTZUG KOMBINIERT MIT IgG ANTIKÖRPER
TRAITEMENT COMBINANT UNE DEPLETION FERRIQUE ET UN ANTICORPS IgG

(30) Priority: 29.04.1993 US 54679
(43) Date of publication of application: 28.02.1996
(73) Proprietor: UNIVERSITY OF IOWA, Iowa City, IA 52242 (US)
(72) Inventor: KEMP, John D., Iowa City, IA 52246 (US)
(74) Representative: Jacob, Reuben Ellis
(86) International application number: US9400911
(87) International publication number: WO94025066

(56) References cited:
- PATHOBIOLOGY (1992), 60(1), 27-32, 1992, XP000608207 KEMP, JOHN D. ET AL: "Effects of anti- transferrin receptor antibodies on the growth of neoplastic cells"
- CANCER RES 52 (15). 1992. 4144-4148, XP002016431 KEMP J D ET AL: "INHIBITION OF HEMATOPOIETIC TUMOR GROWTH BY COMBINED TREATMENT WITH DEFEROXAMINE AND AN IGM MONOCLONAL ANTIBODY AGAINST THE TRANSFERRIN RECEPTOR EVIDENCE FOR A THRESHOLD MODEL OF IRON DEPRIVATION TOXICITY."
- 1992 MEETING OF THE FEDERATION OF AMERICAN SOCIETIES FOR EXPERIMENTAL BIOLOGY (FASEB), PART II, ANAHEIM, CALIFORNIA, USA, APRIL 5-9, 1992. FASEB (FED AM SOC EXP BIOL) J 6 (5). 1992. A2059, XP002016432 KEMP J D ET AL: "INHIBITION OF GROWTH OF MURINE AND HUMAN TUMORS BY COMBINED TREATMENT WITH IGG MABS AGAINST THE TRANSFERRIN RECEPTOR AND DEFEROXAMINE EVIDENCE FOR A CRITICAL THRESHOLD MODEL OF IRON DEPRIVATION TOXICITY."
- Blood, Volume 76, Number 5, issued 01 September 1990, KEMP et al., "Synergistic Inhibition of Lymphoid Tumor Growth in Vitro by Combined Treatment with the Iron Chelator Deferoxamine and an Immunoglobulin G Monoclonal Antibody Against the Transferrin Receptor", pages 991-995, see page 991.
- Journal of the National Cancer Institute, Volume 81, Number 16, issued 16 August 1989, TAETLE et al., "Combination Iron Depletion Therapy", pages 1229-1235, see entire document.
- The Journal of Immunology, Volume 138, Number 8, issued 15 April 1987, KEMP et al., "Role of Transferrin Receptor in Lymphocyte Growth: A Rat IGG Monoclonal Antibody Against the Murine Transferrin Receptor Produces Highly Selective Inhibition of T and B Cell Activation Protocols", pages 2422-2426, see entire document.
- Molecular and Cellular Biology, Volume 5, Number 8, issued August 1985, LESLEY et al., "Inhibition of Cell Growth by Monoclonal Anti-transferrin Receptor Antibodies", pages 1814-1821, see entire document.
- Blood, Volume 69, Number 3, issued March 1987, ESTROV et al., "In Vitro Effects of Deferoxamine in Neonatal Acute Leukemia", pages 757-761, see entire document.
- Cancer Research, Volume 49, Number 49, issued 01 March 1989, CHITAMBAR et al., "Modulation of Lymphocyte Proliferation and Immunoglobulin Production by Transferrin-Gallium", pages 1125-1129, see entire document.
- Proceedings of the National Academy of Sciences, Volume 86, Number 24, issued December 1989, HALLAWAY et al., "Modulation of Deferoxamine Toxicity and Clearance by Covalent Attachment to Biocompatible Polymers", pages 10108-10112, see entire document.

## Description

### TECHNICAL FIELD

The present invention relates to a novel combination of agents for use in inhibiting tumour growth. More specifically, the invention relates to the combination of an antibody and an agent which depletes intracellular iron and works synergistically to prevent tumour growth and can thereby be broadly. applicable in cancer therapy.

### BACKGROUND OF THE INVENTION

Iron uptake from exogenous sources is crucial to the growth of normal and neoplastic cells. Lederman et al, Deferoxamine: A reversible S-phase inhibitor of human lymphocyte proliferation. Blood 64:748, 1984. Iron is a necessary component for the maintenance of cellular DNA synthesis. Iron modulates ribonucleotide reductase activity. Lederman et al, Deferoxamine: A reversible S-phase inhibitor of human lymphocyte proliferation. Blood 64:748, 1984. Data from several sources suggest that iron depletion may be a useful strategy in the treatment of neoplasms, particularly those of hematopoietic origin. Estrov et al, In vitro and in vivo effects of deferoxamine in neonatal acute leukemia. Blood 69:757, 1987; Sauvage et al, Effects of monoclonal antibodies that block transferrin receptor function on the in vivo growth of a syngeneic murine leukemia. cancer Res 47:747, 1987; Taetle et al, Combination iron depletion therapy. J. Natl., Cancer Inst. 81:1229, 1989. Studies in vitro have shown that deferoxamine, an iron chelator, removes iron from ferritin and, to a much less extent, from transferrin. Iron in hemoglobin or cytochromes is not removed by deferoxamine. Deferoxamine was used with some encouraging results in the treatment of a case of acute leukemia. Estrov et al, In vitro and in vivo effects of deferoxamine in neonatal acute leukemia. Blood 69:757, 1987. Further studies have shown that a monoclonal IgM anti-transferrin receptor antibody had significant efficacy in the treatment of murine lymphoma in vivo. Sauvage et al, Effects of monoclonal antibodies that block transferrin receptor function on the in vivo growth of a syngeneic murine leukemia. Cancer Res 47:747, 1987. Further, a combination of the iron chelator parabactin and the multivalent IgA anti-transferrin receptor antibody 42/6 was shown to have supra-additive growth inhibitory effects against the HL60 cell line in vitro. Taetle, Combination iron depletion therapy. J. Natl. Cancer Inst. 81:1229, 1989. The use of an anti-transferrin receptor antibody and iron chelator has been recognized as possibly being an attractive therapeutic strategy because it could potentially allow the utilization of lower doses of a potentially toxic chelator. Kaplinsky et al, Deferoxamine (Desferal)^E-induced ocular toxicity. Ped Hemat Oncol 5:293, 1988. Accordingly, the inventor of the present application has been studying the effects of combination treatment of anti-transferrin receptor antibodies and an iron chelator on the growth of several hematopoietic tumors in vitro.

Taetle et al, in Cancer Research 46: 1759-1763, April 1986, disclosed uncertainty about how multivalent IgA and IgM anti-transferrin receptor antibodies actually work. The 1986 paper proposes that receptor degradation and inhibition of receptor internalization by cross-linking both occur but their more recent work emphasizes the significance of surface cross-linking as a dominant mechanism. Unlike this cross-linking mechanism of action of IgA or IgM, Lesley et al in Mol. Cell. Bio. 5:1814, 1985 provide evidence that IgG anti-transferrin antibodies cause receptor down-regulation by themselves, but do not inhibit tumor growth unless further cross-linked by anti-immunoglobulin antibodies. Their paper discloses that IgM antibodies appear to cross-link when receptor density is high enough and cause growth inhibition. Thus, Lesley et al postulate that extensive cross-linking is required for significant growth inhibition of tumor cells.

While both Taetle et al and Lesley et al do not dispute the concept that IgG anti-transferrin receptor antibodies cause enhanced receptor internalization and degradation, they do suggest that IgG anti-transferrin antibodies are poor growth inhibitors.

Weissman et al in The Journal of Cell Biology, 102:951-958, 1986 provide further evidence that IgG anti-transferrin receptor antibodies cause down-modulation and degradation. Growth inhibition is not addressed in the Weissman et al paper.

Pathobiology (1992), 60(1), p.27-32, Kemp, J.D. *et al*., Cancer Res. 52(15), 1992, pp 4144-4148, Kemp, J.D. *et al*. and 1992 Meeting of the Federation of American Societies for Experimental Biology (FASEB), Part II, Anaheim, California, U.S.A. April 5th to 9th, 1992, Faseb (Fed. Am. Soc. Exp. Biol.) J6(5), 1992, page A2059, Abstract no. 6508 disclose inhibition of lymphoid (hematopoietic) tumour growth *in vitro* by combined treatment with deferoxamine, which is an iron chelator, and an immunoglobulin G (IgG) monoclonal anti-transferrin antibody (ATRA). The first of these prior art documents also suggests the use of gallium to create iron-deprivation and in combination with ATRA to inhibit tumour growth.

Researchers have utilized combined multivalent IgA or IgM anti-transferrin receptor antibodies in combination with iron chelators. However, even though IgA, IgM and IgG are all antibodies, it was expected that IgG would be ineffective in a combination therapy with iron chelator to inhibit tumour growth. This conclusion was drawn from the evidence showing that IgG cannot cross-link surface receptors. Unexpectedly, the inventors of the present invention have found significant synergism in the effects of combination treatment with anti-transferrin receptor IgG antibodies and depletors of intracellular iron on the growth of tumours.

Unlike methods utilizing antibodies which cross-link surface receptors, it is proposed that the IgG anti-transferrin receptor antibodies cause receptor down modulation and are almost as potent an inhibitor as multivalent IgM anti-transferrin receptor antibodies while potentially providing further clinical significance.

### SUMMARY OF THE INVENTION

In accordance with a first aspect of the present invention there is provided use of either (a) deferoxamine or deferoxamine chemically coupled to hydroxyethyl starch (HMW-DFO) or (b) transferrin-gallium for preparation of a medicament for use in an *in vivo* method of inhibiting tumour growth, said method including depleting intracellular iron levels of tumour cells to increase expression of cellular transferrin receptor in the tumour cells by exposing the tumour cells to the deferoxamine or the HMW-DFO or transferrin gallium, and then exposing the tumour cells to monoclonal IgG anti-transferrin receptor antibody (ATRA).

In accordance with a second aspect of the present invention there is provided use of monoclonal IgG anti-transferrin receptor antibody (ATRA) for preparation of a medicament for use in an *in vivo* method of inhibiting tumour growth, said method including depleting intracellular iron levels of tumour cells to increase expression of cellular transferrin receptor in the tumour cells by exposing the cells to either (a) deferoxamine or deferoxamine chemically coupled to hydroxyethyl starch (HMW-DFO) or (b) transferrin-gallium and then exposing the cells to said monoclonal IgG anti-transferrin receptor antibody.

In accordance with a third aspect of the present invention there is provided use of either (a) deferoxamine or deferoxamine chemically coupled to hydroxyethyl starch (HMW-DFO) or (b) transferrin gallium, and monoclonal IgG anti-transferrin receptor antibody for preparation of a medicament or medicaments for use in an *in vivo* method of inhibiting tumour growth, said method including depleting intracellular iron levels of tumour cells to increase expression of cellular transferrin receptor in the tumour cells by exposing the tumour cells to the deferoxamine or the HMW-DFO or the transferrin-gallium and then exposing the tumour cells to the monoclonal IgG anti-transferrin receptor antibody (ATRA).

In accordance with further aspects of the present invention there are provided:

Use of deferoxamine or deferoxamine chemically coupled to hydroxyethyl starch (HMW-DFO) for preparation of a medicament for use in *in vivo* method of inhibiting thymidine incorporation into tumour cells, said method including: depleting intracellular iron levels of the tumour cells to inhibit thymidine incorporation by exposing the tumour cells to the deferoxamine or HMW-DFO, and exposing the tumour cells to monoclonal IgG anti-transferrin receptor antibody and enhancing the inhibitory effect.

Use of monoclonal IgG anti-transferrin receptor antibody for preparation of a medicament for use in an *in vivo* method of inhibiting thymidine incorporation into tumour cells, said method including: depleting intracellular iron levels of the tumour cells to inhibit thymidine incorporation by exposing the tumour cells to deferoxamine or deferoxamine chemically coupled to hydroxyethyl starch (HMW-DFO), and exposing the tumour cells to monoclonal IgG anti-transferrin receptor antibody and enhancing the inhibitory effect.

Use of deferoxamine or deferoxamine chemically coupled to hydroxyethyl starch (HMW-DFO), and monoclonal IgG anti-transferrin receptor antibody for preparation of a medicament or medicaments for use in an *in vivo* method of inhibiting method of inhibiting thymidine incorporation into tumour cells, said method including: depleting intracellular iron levels of the tumour cells to inhibit thymidine incorporation by exposing the tumour cells to the deferoxamine or HMW-DFO, and exposing the tumour cells to the monoclonal IgG anti-transferrin receptor antibody and enhancing the inhibitory effect.

Use of a deferoxamine derivative which is deferoxamine chemically coupled to hydroxyethyl starch (HMW-DFO) for preparation of a medicament for use in an *in vivo* method of eliminating tumour growth, said method including depleting intracellular iron levels of tumour cells to increase expression of cellular transferrin receptor in the tumour cells by exposing the tumour cells to the HMW-DFO, and exposing the tumour cells to monoclonal IgG anti-transferrin receptor antibody.

Use of monoclonal IgG anti-transferrin receptor antibody for preparation of a medicament for use in an *in vivo* method of eliminating tumour growth, said method including depleting intracellular iron levels of tumour cells to increase expression of cellular transferrin receptor in the tumour cells by exposing the tumour cells to a deferoxamine derivative which is deferoxamine chemically coupled to hydroxyethyl starch (HMW-DFO), and exposing the tumour cells to the monoclonal IgG anti-transferrin receptor antibody.

Use of a deferoxamine derivative which is deferoxamine chemically coupled to hydroxyethyl starch (HMW-DFO) and monoclonal IgG anti-transferrin receptor antibody for preparation of a medicament for use in an *in vivo* method of eliminating tumour growth, said method including depleting intracellular iron levels of tumour cells to increase expression of cellular transferrin receptor in the tumour cells by exposing the tumour cells to the HMW-DFO, and exposing the tumour cells to the monoclonal IgG anti-transferrin receptor antibody.

### FIGURES IN THE DRAWINGS

Other advantages of the present invention will be readily appreciated as the same becomes better understood by reference to the following detailed description when considered in connection with the accompanying drawings wherein:

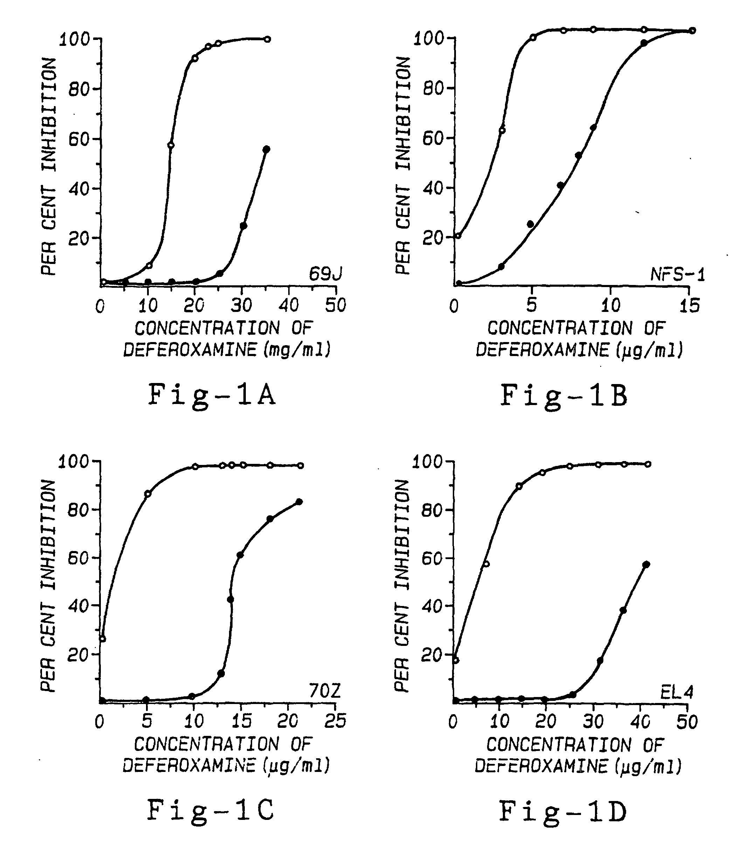

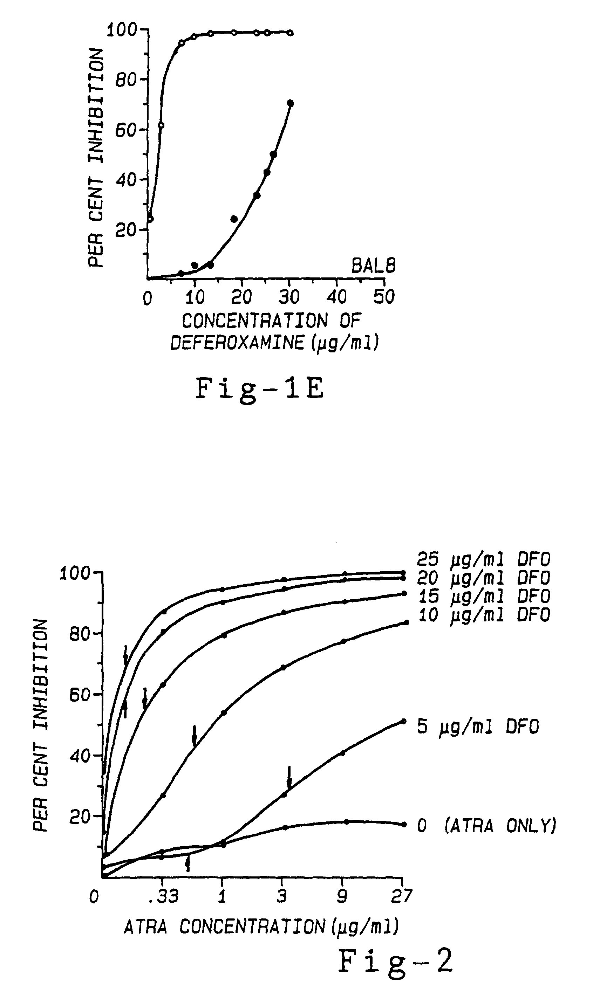

Figure 1 shows the effect of combined treatment with deferoxamine (DFO) and the IgG anti-transferrin receptor antibody (ATRA) C2F2 on tumor cell growth. Five lymphoid tumors were cultured with increasing doses of DFO, with (open circles) or without (closed circles) the presence of ATRA at 25ug/ml, as described in the Materials and Methods portion of the Experimental Findings Section of the present application. The data are presented as percent inhibition of thymidine incorporation; the decreases in thymidine incorporation were closely related to decreases in viable cell yields in three experiments (not shown). The control levels of incorporation were 217,126 cpm (69J), 546,036 cpm (NFS), 491,648 cpm (70Z), 490,190 cpm (EL4), and 428,943 cpm (Bal8). Normal rat IgG at 25 ug/ml had no significant effect at several concentrations of DFO with each tumor. Very similar results were obtained in a repetition of the same protocol and have also been repeatedly observed for each of the five tumors in subsequent related experiments.

Figure 2 shows two-way dose/response analysis of the effects of DFO and an IgG ATRA on growth of the EL4 tumor. Cells were cultured as noted in Materials and Methods and were exposed to varying concentrations of the ATRA for each of the noted doses of DFO. Percent inhibition of thymidine incorporation was determined from a tumor alone control of 456,992 cpm. Arrows denote half-maximal inhibition points for each curve. Very similar results were obtained in a repetition of the same protocol.

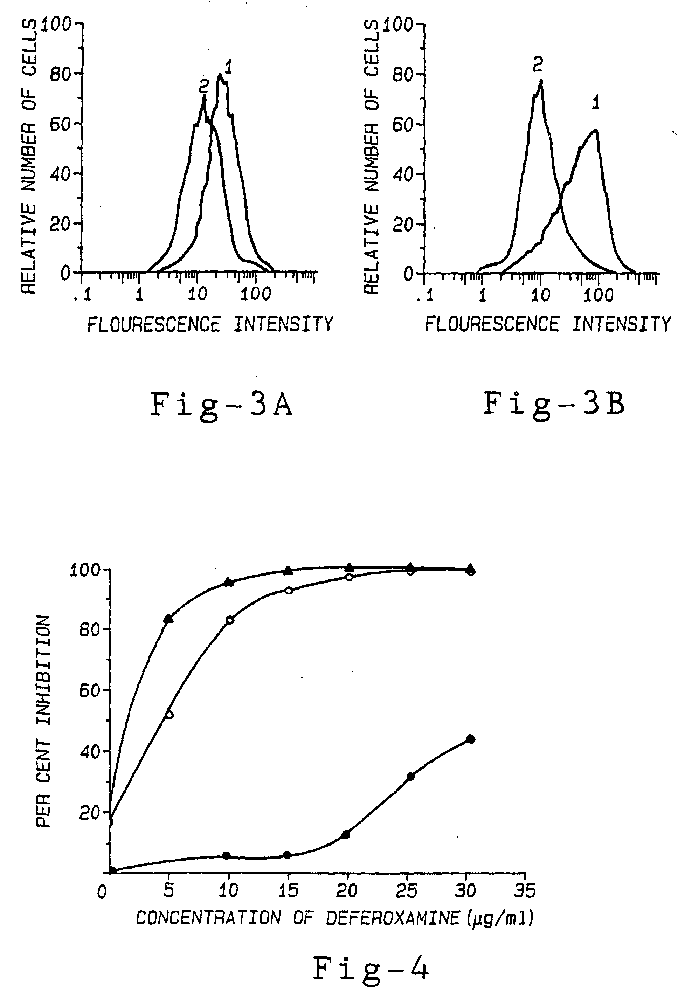

Figure 3 shows flow cytometric analysis of the effects of DFO and an IgG ATRA on surface expression of the transferrin receptor of NFS cells. Cells were cultured for 48 hours as noted in Materials and Methods and were exposed to DFO, the IgG ATRA, or a combination of the two. Panel A., curve 1 represents data obtained from cells in the presence of normal rat IgG while curve 2 represents cells exposed to the IgG ATRA (both reagents at 25 ug/ml). Panel B, curve 1 represents cells exposed to DFO alone (5ug/ml) while curve 2 represents cells exposed to DFO and the ATRA (5 and 25 ug/ml, respectively). In the same experiment, lower doses of DFO alone produced smaller increases in surface expression of the transferrin receptor, while higher doses produced only slightly higher increases; the combination of DFO/ATRA produced receptor down-modulation at all doses of DFO (not shown). Very similar data were obtained in two repetitions of the same protocol with the NFS tumor, and related studies have shown that the other four tumors also show receptor down-modulation when treated with DFO and the IgG ATRA.

Figure 4 shows a comparison of the enhancement of inhibition produced by an IgG ATRA to that produced by an IgM ATRA, when both are used in combination with DFO. EL4 cells were cultured as noted in Materials and Methods. The curved marked by closed circles represents the effect of DFO alone; that marked by open circles represents the effect of DFO and the IgG ATRA C2F2; and that marked by closed triangles represents the effect of DFO and the IgM ATRA R17-208. Both ATRAS were used at a concentration of 25 ug/ml. Very similar data were obtained in a repetition of the same protocol.

### DETAILED DESCRIPTION OF THE INVENTION

The present invention presents a combination of agents for inhibiting tumor growth by depleting intracellular iron levels of tumor cells and then exposing the cells to monoclonal IgG anti-transferrin receptor antibody. The depletion of intracellular iron levels of the tumor cells increases expression of cellular transferrin receptors in the tumor cells, as detailed in the many papers cited in the background section.

Cells can be depleted of intracellular iron by several methods, such as exposing the cells to an iron chelator, such as deferoxamine (DFO) and deferoxamine chemically coupled to hydroxyethyl starch (the bound combination is known as high molecular weight deferoxamine (HMW-DFO)) which is available from Biomedical Frontiers of Minneapolis or parabactin. The coupling or covalent attachment of deferoxamine to a biocompatible polymer such as hydroxyethyl starch is disclosed in Hallaway et al., Modulation of deferoxamine toxicity and clearance by covalent attachment to biocompatible polymers, Proc. Natl. Acad. Sci USA, 86:10108-10112, 1989. DFO, as an exemplary iron chelator, is available as deferoxamine mesylate. It has been used clinically in acute iron poisoning. However, DFO causes a number of allergic reactions, including pruritus, wheals, rash, and anaphylaxis. It is therefore advantageous in combined therapy to be able to use a synergistic agent which permits a lower dose of the chelator thereby requiring a dose significantly below the toxic blood level of the iron chelator.

Alternatively, cellular iron levels can be depleted by the exposure of the cells to gallium, as in the form of transferrin-gallium. Chitambar et al, J. Clin. Invest. 78, 1538-1546, December 1986, and Foster et al, Cancer Treatment Reports 70:1311-1319, November 1986, disclose the anti-tumor activity of gallium. The Chitambar et al paper suggests that transferrin-gallium impairs intercelluar release of 59Fe from transferrin by interfering with the process responsible for intracellular acidification. Accordingly, transferrin-gallium and possibly other gallium compounds may be suitable for use with the present invention.

Once the tumor cells are partially depleted of intracellular iron, the expression of cellular transferrin receptor in the cells is increased. The tumor cells are then exposed to monoclonal IgG ATRA. IgG ATRA can be biologically derived by methods well known in the art. Alternatively, equivalents of IgG ATRA which are genetically engineered can be used in accordance with the present invention. Examples of genetically engineered equivalents of IgG are disclosed by Sastry et al, Cloning of the immunological repertoire in E. coli for generation of monoclonal catalytic antibodies: Construction of a heavy chain variable region-specific cDNA library. Proc. Natl. Acad. Sci. 86:5728-4732, 1989; Huse, et al, Generation of a large combinatorial library of the immunoglobulin repertoire in phage lambda. Science 246:1275-1281, 1989.

The following data support the contention that the combination of the iron depletion resulting in increased transferrin receptor expression with the effect of the IgG antibody to cause transferrin receptor down modulation and degradation results in the significant synergistic effect of the combined therapy to inhibit tumor growth.

Although the data below have been compiled for hematopoietic cells, it is very likely that the present combination therapy would also be effective in solid tumors, possibly to a greater extent than combined therapy using IgM or IgA with iron chelators. This is because the IgG antibody diffuses into interstitial fluids much more freely than IgM or IgA antibodies and IgG antibodies are therefore much more likely to be able to affect tumor cells outside of the vascular spaces.

The following data illustrate the effects of the combination treatment with monoclonal IgG anti-transferrin receptor antibodies and an iron chelator on the growth of several hematopoietic tumors both in vitro and in vivo. The data characterize quantitatively and mechanistically the effects of the combined therapy.

### EXPERIMENTAL FINDINGS

### Materials and Methods

### IN VITRO STUDY - DEFEROXAMINE

Lymphoid tumors. The B-cell tumors 702, NFS-1, and 69J were obtained from Drs. Bryan Van Ness (now at the University of Minnesota), Herbert Morse (NIH), and Richard Lynch (University of Iowa), respectively. The T-cell tumors Bal8 and EL4 were also obtained from Dr. Lynch. Each has been maintained in the laboratory of the inventor for several years.

Deferoxamine. The bacterial iron siderphore deferoxamine (produced under the name Desferal by Ciba-Geigy Inc., Sumitt, NJ) was obtained from the Pharmacy of the University of Iowa Hospitals and Clinics. A fresh vial containing 500 mg of lyophilized deferoxamine was used for each experiment and was resuspended with distilled water to a 100mg/ml stock solution before dilution in tissue culture media.

Anti-transferrin receptor antibodies (ATRAs). The details of the derivation of the IgG2a rat anti-mouse transferrin receptor antibody C2F2 have been previously published. Kemp et al, Role of the transferrin receptor in lymphocyte growth: A rat IgG monoclonal antibody against the murine transferrin receptor produces highly selective inhibition of T and B cell activation protocols. J Immunol 138:2422, 1987. C2F2 and the IgM ATRA R17-208, obtained from the American Type Culture Collection (ATCC) (TIB 220), were purified as previously described. Kemp et al, Inhibition of lymphocyte activation with anti-transferrin receptor Mabs: A comparison of three reagents and further studies of their range of effects and mechanism of action. Cell Immunol 122:218, 1989.

Culture conditions. For all of the experiments, each of the tumors were cultured with a starting density of 1.25 x 105 cells/ml. The tissue culture media consisted of RPMI-1640 supplemented with 10% FCS. 0.1 mM nonessential amino acids, 1.0 mM sodium pyruvate, 2.0 mM L-glutamine, 100 U/ml penicillin, 100 ug/ml streptomycin, 10mM hepes, and 5 x 10-3 M 2ME. The cultures that were assayed for thymidine incorporation were carried out in triplicate in 200ul volumes in 96 well flat-bottom plates (Corning or Costar), and were harvested for scintillation counting after a pulse with 1 uC/well of [3H] thymidine (Amersham, Arlington Heights, IL) during the last four or six hours of a standard 48-hour culture period. Standard deviations of triplicate cpm data were, with rare exceptions, less than 5% of group means. Cultures that were harvested for FACS analysis were carried out in T-25 or T-75 flasks.

Flow cytometric analysis. Cells harvested from culture were stained with C2F2 and a fluorescein conjugated F(ab')2 preparation of mouse anti-rat IgG (Jackson Immunoresearch, West Grove, PA). In those cases where C2F2 was included in the culture, we (and others) have shown that most of the transferrin receptors are already bound by the ATRA and this can be detected by the addition of the secondary reagent only. Nevertheless, a small additional increment in staining intensity is routinely observed when a post-culture exposure to the ATRA is included, and this has become the inventor's standard practice. Kemp et al, Inhibition of lymphocyte activation with anti-transferrin receptor Mabs: A comparison of three reagents and further studies of their range of effects and mechanism of action. Cell Immunol 122:218, 1989). The analysis was carried out on a FACS 440 (Becton-Dickinson) equipped with four decade logarithmic signal amplifiers. Data were processed and plotted with the Electric Desk software run on a Dec Vax 750 computer.

### IN VITRO RESULTS - DEFEROXAMINE

Each tumor to be tested was evaluated beforehand with respect to its sensitivity to deferoxamine.

The data in Figure 1 show that the inhibition of thymidine incorporation produced by submaximal concentrations of DFO is dramatically enhanced in the presence of the IgG ATRA for each of the five tumors tested. The ATRA alone, even at the relatively high concentration of 25 ug/ml, produces only modest inhibition - ranging from 0 to 26% depending upon the tumor in question. That IgG ATRAs, by themselves, are poor inhibitors of tumor cell growth in vitro is entirely consistent with prior studies by others (Lesley et al, Inhibition of cell growth by monoclonal anti-transferrin receptor antibodies. Mol Cell Biol 5:1814, 1985; Taetle et al, Mechanisms of growth inhibition by anti-transferrin receptor monoclonal antibodies. Cancer Res 46:1759, 1986).

Although at certain doses of DFO the effect of the reagents combined produces very impressive synergistic inhibition, a more conservative way to gauge the interaction of the two reagents is to compare the estimated dose of DFO required to produce half-maximal inhibition in the presence of the ATRA versus that required in the absence of the ATRA, to establish an index of enhancement by dividing the latter number by the former. The numerator, in all cases, takes into account the baseline inhibition caused by the ATRA itself. The indices thereby obtained for each tumor are 2.5 (69J), 2.5 (NFS), 4.0 (70Z), 5.3 (EL4), and 8.8 (Bal8), for the experiment shown. A second experiment yielded indices of 2.0 (69J), 2.5 (NFS), 3.0 (70Z), 4.0 (EL4), and 5.0 (Bal8); subsequent related experiments have further confirmed these observations. It appears that all of the tumors tested show a reproducible synergistic enhancement of inhibition when the two agents are used in combination, and that certain tumors reproducibly show more enhancement than others.

The dose range of the ATRA required to produce synergistic inhibition was determined. The data are presented in Figure 2. At the very lowest dose of DFO employed (5 ug/ml), the amount of ATRA required to produce half-maximal inhibition is greater than that required in the absence of DFO. This result would be expected from a simple increase in target antigen density if all other factors remained unchanged. However, with each subsequent increase in the does of DFO the amount of ATRA required to produce half-maximal inhibition declines to an apparent minimum of about 100 to 200 ng/ml. The latter observation suggests that as the dose of DFO increases, so does the inhibitory efficiency of the ATRA.

The mechanisms involved in IgG ATRA/DFO synergism were characterized. Flow cytometric studies were conducted of changes in surface expression of the transferrin receptor resulting from the treatments under study.

The data represented in panel A of Figure 3 show that the IgG ATRA alone causes about a three-fold drop in surface receptor expression when compared to the control. This result was expected from prior work. Lesley et al, Inhibition of cell growth by monoclonal anti-transferrin receptor antibodies. Mol Cell Biol 5:1814, 1985; Weissman et al, Exposure of K562 cells to anti-receptor monoclonal antibody OKT9 results in rapid redistribution and enhanced degradation of the transferrin receptor. J. Cell. Bio. 102:951, 1986. The data represented in panel B of Figure 3 show that DFO alone causes a three-fold increase in surface receptor expression (compare curve 1 in panel B to curve 1 in panel A). This result was also expected from prior work. Rao et al, Effects of alterations in cellular iron on biosynthesis of the transferrin receptor in K562 cells. Mol Cell Biol 5:595, 1985. Curve 2 in panel B shows the effect of the reagent combination. It is clear that ATRA-induced receptor down-modulation persists and dominates in spite of the presence of a stimulus for enhanced receptor expression.

It has generally been observed that IgG ATRAs, as single agents, are poor inhibitors of tumor cell growth and the data presented here continue to support that view. Therefore, having made the unexpected observation that an IgG ATRA can cause synergistic growth inhibition when used with an iron chelator, growth inhibition was compared with that produced by an IgM ATRA in combination with DFO. For the comparison, an IgM ATRA was chosen that has recently been shown to have anti-tumor effects in vivo. Sauvage et al, Effects of Monoclonal Antibodies that Block Transferrin Receptor Functions on the In Vivo Growth of a Syngeneic Murine Leukemia. Cancer Res. 47:747, 1987. The data presented in Figure 4 show that the IgM ATRA is only modestly more effective than the IgG ATRA in that 99% inhibition of thymidine incorporation occurs at a slightly lower concentration of DFO.

Nevertheless, both ATRAs produce 99% inhibition at concentrations of DFO that would otherwise have only very modest effects. The relative inhibitory potencies of the IgM and IgG ATRAS in these experiments are quite similar to those seen in normal lymphocyte activation protocols. Kemp et al, Inhibition of lymphocyte activation with anti-transferrin receptor Mabs: A comparison of three reagents and further studies of their range of effects and mechanisms of action. Cell Immunol 122:218, 1989.

The above data confirm and significantly extend the work recently reported by Taetle et al, Combination iron depletion therapy. J. Natl., Cancer Inst. 81:1229, 1989. Taetle et al reported superadditive inhibition of HL60 growth in vitro when that tumor was exposed to a combination of an iron chelator parabactin and the multivalent IgA ATRA 42/6. The present invention provides an advancement by showing that synergistic inhibition of tumor growth also occurs. In all cases tested, 5 murine lymphoid tumors are exposed to deferoxamine and the monoclonal IgG anti-transferrin receptor C2F2 in vitro. The results were unexpected because while it has been shown that monoclonal IgG anti-transferrin receptor antibodies can act as potent inhibitors of normal lymphocyte activation, such reagents have nonetheless consistently failed to produce significant inhibition of tumor cell growth when used alone. Kemp et al, Inhibition of lymphocyte activation with anti-transferrin receptor Mabs: A comparison of three reagents and further studies of their range of effects and mechanisms of action. Cell Immunol 122:218, 1989; Lesley et al in Mol. Cell. Bio. 5:1814, 1985; and Taetle et al, Combination iron depletion therapy. J. Natl., Cancer Inst. 81:1229, 1989;

The above data provide further support for the generally accepted view that monoclonal IgG anti-transferrin receptor antibodies impair cell function by causing down-modulation and degradation of the transferrin receptor, thereby impairing the delivery of iron. This occurs even when surface receptor density is increased by treatment with the deferoxamine. Moreover, the antibody dose response curve converts into a pattern that shows a progressive shift to the left, rather than continuing the shift to the right with increasing doses of deferoxamine. This indicates that the antibody actually becomes a more efficient inhibitor. It can be hypothesized that DFO increases the turnover velocity and/or alters the intracellular traffic pattern of transferrin receptors. Either may allow for increased iron uptake, but each one might also increase the risk that an antibody/receptor complex would be diverted to a lysosome and degraded.

### IN VIVO STUDY - DEFEROXAMINE

### Materials and Methods

To demonstrate the in vivo utility of the invention, four groups of C3H/HEJ mice were injected intradermally with 50,000 cells of histocompatible 38C13 B-cell lymphoma and observed for 11 days. One experimental Group (A) received deferoxamine only. This group received the deferoxamine by subcutaneous infusion pump and intramuscular injection. A second Group (B) was given saline via infusion pumps. A third Group (C) was given injections of monoclonal anti-transferrin receptor antibody C2F2 without deferoxamine. Finally, the fourth Group (D) received combined treatment with DFO and the monoclonal anti-transferrin receptor antibody.

Tumor weight, in milligrams, was measured and estimated by a previously reported technique (Cancer Chemother. Rep., Part 3, 3:18-55, 1972).

### IN VIVO RESULTS DEFEROXAMINE

The tumor weight measured and estimated as described above was as follows:

| | |
|---|---|
| Group A | 1,761±68(±1 S.D.) |
| Group B | 1,396±722 |
| Group C | 342±128 |
| Group D | 172±45 |

Groups A and B are not significantly different by t-test, thus indicating that DFO treatment alone had no effect on tumor growth. Group C is significantly different from either Group A or Group B at the .001 level, thus indicating that treatment with the IgG anti-transferrin receptor antibody inhibited tumor growth. Finally, Group D is significantly different from Group C at the .05 level, thus indicating that combined treatment with DFO and the monoclonal antibody provided yet a further reduction in tumor growth. The latter result indicates synergy because DFO alone had no detectable effect.

In a second related experiment, the combined treatment group (equivalent to Group D in the first experiment) included two mice which showed no detectable tumor growth at all. Moreover, an additional control group for the injection of the monoclonal antibody consisting of injection of an equal concentration of normal rat IgG showed no inhibition of tumor growth. This was the expected result and provides a strong indication that the anti-transferrin receptor antibody effect is specific.

These experiments demonstrate the in vivo utility of the findings previously obtained in vitro (Blood 76:991-995, 1990). Moreover, they are more rigorous and relevant to potential human therapy because the artificial nude mouse/human tumor xenograph model has not been employed. In this experiment, a normal mouse served as host for a mouse tumor and was treated with an anti-mouse transferrin receptor antibody. Thus, normal tissue expression of the transferrin receptor is taken into account with respect to the possibility that normal tissue might bind antibody and make it less effective for tumor therapy.

The antibody has been effective and it has interacted with DFO, as expected from the in vitro studies. This is unambiguous evidence of in vivo utility of the concept put forth based on the in vitro studies.

### Materials and Methods - Use of HMW-DFO In Vivo

The experiments were conducted in nearly identical manner to the in vivo studies with deferoxamine previously discussed above. That is, 50,000 cells of 38C13 lymphoma were given intradermally to C3H mice in all groups.

As in the Deferoxamine experiments, the IgG ATRA was administered in 3 mg. doses on days 0, 3, 6, and 9. The experiments were terminated on day 11. HMW-DFO was given as a single daily intraperitoneal injection of 0.5 ml. of a 26mM solution. The starch control was administered in a similar manner (0.5 ml i.p.of a 10% solution daily). An additional control in the second experiment (not shown) utilized normal rat IgG in the same concentrations and dose schedule as C2 and the result was not significantly different than the untreated group (ATW of .44g-96% of control).

### RESULTS - HMW-DFO

The treatment groups and results were as follows:

| EFFECTS ON TUMOR GROWTH: HMW-DFO, IGG ATRA, OR BOTH | | | | | |
|---|---|---|---|---|---|
| GRP | Rx | AVG TUM WGT (EXP1) | %CON AVG | TUM WGT (EXP2) | %CON |
| 1. | Untr.Con | .57g | 100% | .46g | 100% |
| 2. | HMW-DFO | .69g | 121% | .59g | 128% |
| 3. | Starch Con. | .69g | 121% | .55g | 120% |
| 4. | HMW+C2 | .00g | 0.0% | .00g | 0.0% |
| 5. | C2 only | .33g | 58% | .09g | 20% |
| 6. | Starch+C2 | .48g | 84% | .26g | 57% |

All percent values are relative to the untreated control group. There is a slight enhancement in tumor growth in groups 1 and 2 that may be due to the starch polymer. The ineraction between HMW-DFO and the ATRA can clearly be called synergistic in both experiments since the HMW-DFO has no inhibitory effect by itself. There is complete inhibition of tumor growth in all animals receiving HMW-DFO and the IgG ATRA in both experiments. There is no apparent toxicity. This is further unambiguous evidence of the in vivo utility of the concept put forth.

It is believed that the capacity to cause inhibition of tumor cell growth when used in combination with a depletor of intracellular iron will be a feature common to most, if not all, IgG anti-transferrin receptor antibodies. Preliminary comparative studies have been conducted with two other IgG anti-transferrin rat antibodies to date and both show synergy with deferoxamine. The mouse anti-human transferrin receptor antibody OKT9 has shown synergy against HL60 and the rat anti-mouse transferrin receptor YE1 has shown synergy against EL4. This apparent similarity between IgG anti-transferrin receptor with respect to their synergy with deferoxamine is similar to their demonstrable similarity in other functional assays. Prior studies that were performed by the inventor of the present invention indicated that three IgG anti-transferrin receptors antibodies exhibit similar profiles of inhibitory capacities in a variety of normal lymphocyte activation protocols. Kemp et al, Inhibition of lymphocyte activation with anti-transferrin receptor Mabs: A comparison of three reagents and further studies of their range of effects and mechanisms of action. Cell Immunol 122:218, 1989. The same prior studies showed, in addition, that an IgM anti-transferrin receptor antibody had only a modest advantage as in an inhibitor in vitro. This prior data is consistent with the data presented herein. From a therapeutic point of view, the latter finding has real potential significance because IgG antibodies diffuse into interstitial fluids much more freely than IgM or IgA antibodies. Accordingly, IgG antibodies are more likely to be able to affect tumor cells outside of vascular spaces.

### REFERENCES/PUBLICATIONS CITED

1. Blood: 64:748, 1984 (Lederman)
2. Blood: 69:757, 1987 (Estrov)
3. Cancer Res 47:747, 1987 (Sauvage)
4. J.Natl., Cancer Inst. 81:1229, 1989 (Taetle)
5. Ped Hemat Oncol 5:293, 1988 (Kaplinsky)
6. Cancer Research 46:1759-1763, April 1986 (Taetle)
7. Mol. Cell. Bio. 5:1814, 1985 (Lesley)
8. The Journal of Cell Biology, 102:951-958 (Weissman)
9. Proc. Natl. Acad. Sci USA, 86:10108-10112, 1989 (Hallaway)
10. J. Clin. Invest. 78, 1538-1546, December 1986 (Chitambar)
11. Cancer Treatment Reports 70:1311-1319, November 1986 (Foster)
12. Proc. Natl. Acad. Sci 86:5728-5732, 1989 (Sastry)
13. Science 246:1275-1281, 1989 (Huse)
14. J. Immunol 138:2422, 1987 (Kemp)
15. Cell Immul 122:218, 1989 (Kemp)
16. Mol Cell Biol 5:595, 1985 (Rao)
17. Cancer Chemother. Rep., Part 3, 3:18-55, 1972)
18. Blood 76:991-995, 1990

## Claims

1. Use of either (a) deferoxamine or deferoxamine chemically coupled to hydroxyethyl starch (HMW-DFO) or (b) transferrin-gallium for preparation of a medicament for use in an *in vivo* method of inhibiting tumour growth, said method including depleting intracellular iron levels of tumour cells to increase expression of cellular transferrin receptor in the tumour cells by exposing the tumour cells to the deferoxamine or the HMW-DFO or transferrin gallium, and then exposing the tumour cells to monoclonal IgG anti-transferrin receptor antibody (ATRA).

2. Use as set forth in claim 1 wherein said method includes increasing the dose of the deferoxamine or HMW-DFO to increase the efficiency of the antibody as a growth inhibitor.

3. Use as set forth in either preceding claim wherein in said method exposure of the tumour cells to the monoclonal IgG ATRA causes transferrin receptor down-modulation and degradation.

4. Use as set forth in any preceding claim wherein the tumour cells are hematopoietic tumour cells.

5. Use as set forth in any of claims 1 to 3 wherein the tumour cells are non-hematopoietic tumour cells.

6. Use of monoclonal IgG anti-transferrin receptor antibody (ATRA) for preparation of a medicament for use in an *in vivo* method of inhibiting tumour growth, said method including depleting intracellular iron levels of tumour cells to increase expression of cellular transferrin receptor in the tumour cells by exposing the cells to either (a) deferoxamine or deferoxamine chemically coupled to hydroxyethyl starch (HMW-DFO) or (b) transferrin-gallium and then exposing the cells to said monoclonal IgG anti-transferrin receptor antibody.

7. Use as set forth in claim 6 wherein said method further includes increasing the dose of the deferoxamine or HMW-DFO to increase the efficiency of the antibody as a growth inhibitor.

8. Use as set forth in either of claims 6 and 7 wherein in said method exposure of the tumour cells to the monoclonal IgG ATRA causes transferrin receptor down-modulation and degradation.

9. Use as set forth in any of claims 6 to 8 wherein the tumour cells are hematopoietic tumour cells.

10. Use as set forth in any of claims 6 to 8 wherein the tumour cells are non-hematopoietic tumour cells.

11. Use of either (a) deferoxamine or deferoxamine chemically coupled to hydroxyethyl starch (HMW-DFO) or (b) transferrin gallium, and monoclonal IgG anti-transferrin receptor antibody for preparation of a medicament or medicaments for use in an *in vivo* method of inhibiting tumour growth, said method including depleting intracellular iron levels of tumour cells to increase expression of cellular transferrin receptor in the tumour cells by exposing the tumour cells to the deferoxamine or the HMW-DFO or the transferrin-gallium and then exposing the tumour cells to the monoclonal IgG anti-transferrin receptor antibody (ATRA).

12. Use as set forth in claim 11 wherein said method further includes increasing the dose of the deferoxamine or the HMW-DFO to increase the efficiency of the antibody as a growth inhibitor.

13. Use as set forth in either of claims 11 and 12 wherein in said method exposure of the tumour cells to the monoclonal IgG ATRA causes transferrin receptor down-modulation and degradation.

14. Use as set forth in any of claims 11 to 13 wherein the tumour cells are hematopoietic tumour cells.

15. Use as set forth in any of claims 11 to 13 wherein the tumour cells are non-hematopoietic tumour cells.

16. Use of deferoxamine or deferoxamine chemically coupled to hydroxyethyl starch (HMW-DFO) for preparation of a medicament for use in *in vivo* method of inhibiting thymidine incorporation into tumour cells, said method including: depleting intracellular iron levels of the tumour cells to inhibit thymidine incorporation by exposing the tumour cells to the deferoxamine or HMW-DFO, and exposing the tumour cells to monoclonal IgG anti-transferrin receptor antibody and enhancing the inhibitory effect.

17. Use of monoclonal IgG anti-transferrin receptor antibody for preparation of a medicament for use in an *in vivo* method of inhibiting thymidine incorporation into tumour cells, said method including: depleting intracellular iron levels of the tumour cells to inhibit thymidine incorporation by exposing the tumour cells to deferoxamine or deferoxamine chemically coupled to hydroxyethyl starch (HMW-DFO), and exposing the tumour cells to monoclonal IgG anti-transferrin receptor antibody and enhancing the inhibitory effect.

18. Use of deferoxamine or deferoxamine chemically coupled to hydroxyethyl starch (HMW-DFO), and monoclonal IgG anti-transferrin receptor antibody for preparation of a medicament or medicaments for use in an *in vivo* method of inhibiting method of inhibiting thymidine incorporation into tumour cells, said method including: depleting intracellular iron levels of the tumour cells to inhibit thymidine incorporation by exposing the tumour cells to the deferoxamine or HMW-DFO, and exposing the tumour cells to the monoclonal IgG anti-transferrin receptor antibody and enhancing the inhibitory effect.

19. Use of a deferoxamine derivative which is deferoxamine chemically coupled to hydroxyethyl starch (HMW-DFO) for preparation of a medicament for use in an *in vivo* method of eliminating tumour growth, said method including depleting intracellular iron levels ,of tumour cells to increase expression of cellular transferrin receptor in the tumour cells by exposing the tumour cells to the HMW-DFO, and exposing the tumour cells to monoclonal IgG anti-transferrin receptor antibody.

20. Use of monoclonal IgG anti-transferrin receptor antibody for preparation of a medicament for use in an *in vivo* method of eliminating tumour growth, said method including depleting intracellular iron levels of tumour cells to increase expression of cellular transferrin receptor in the tumour cells by exposing the tumour cells to a deferoxamine derivative which is deferoxamine chemically coupled to hydroxyethyl starch (HMW-DFO), and exposing the tumour cells to the monoclonal IgG anti-transferrin receptor antibody.

21. Use of a deferoxamine derivative which is deferoxamine chemically coupled to hydroxyethyl starch (HMW-DFO) and monoclonal IgG anti-transferrin receptor antibody for preparation of a medicament for use in an *in vivo* method of eliminating tumour growth, said method including depleting intracellular iron levels of tumour cells to increase expression of cellular transferrin receptor in the tumour cells by exposing the tumour cells to the HMW-DFO, and exposing the tumour cells to the monoclonal IgG anti-transferrin receptor antibody.

## Patentansprüche

1. Verwendung von entweder (a) Deferoxamin bzw. chemisch an Hydroxyethylstärke gekoppeltem Deferoxamin (HMW-DFO) oder von (b) Transferrin-Gallium zur Herstellung eines Arzneimittels für die Verwendung in einem *in-vivo*-Verfahren zur Inhibierung von Tumorwachstum, wobei das Verfahren umfaßt, intrazelluläre Eisenspiegel von Tumorzellen zur Erhöhung der Expression des zellulären Transferrinrezeptors in den Tumorzellen zu verringern, indem die Tumorzellen Deferoxamin bzw. HMW-DFO oder Transferrin-Gallium ausgesetzt werden, und anschließend die Tumorzellen einem monoklonalen IgG-Antitransferrinrezeptor-Antikörper (ATRA) auszusetzen.

2. Verwendung nach Anspruch 1, wobei das Verfahren die Erhöhung der Deferoxamin- bzw. HMW-DFO-Dosis umfaßt, um die Wirksamkeit des Antikörpers als Wachstumsinhibitor zu erhöhen.

3. Verwendung nach einem der vorhergehenden Ansprüche, wobei in dem Verfahren der Transferrinrezeptor heruntermoduliert und abgebaut wird, dadurch daß die Tumorzellen dem monoklonalen IgG-ATRA ausgesetzt werden.

4. Verwendung nach einem der vorhergehenden Ansprüche, wobei es sich bei den Tumorzellen um hämatopoetische Tumorzellen handelt.

5. Verwendung nach einem der Ansprüche 1 bis 3, wobei es sich bei den Tumorzellen um nichthämatopoetische Tumorzellen handelt.

6. Verwendung eines monoklonalen IgG-Antitransferrinrezeptor-Antikörpers (ATRA) zur Herstellung eines Arzneimittels für die Verwendung in einem *in*-*vivo*-Verfahren zur Inhibierung von Tumorwachstum, wobei das Verfahren umfaßt, intrazelluläre Eisenspiegel von Tumorzellen zur Erhöhung der Expression des zellulären Transferrinrezeptors in den Tumorzellen zu verringern, indem die Zellen entweder (a) Deferoxamin bzw. chemisch an Hydroxyethylstärke gekoppeltem Deferoxamin (HMW-DFO) oder (b) Transferrin-Gallium ausgesetzt werden, und anschließend die Zellen dem monoklonalen IgG-Antitransferrinrezeptor-Antikörper (ATRA) auszusetzen.

7. Verwendung nach Anspruch 6, wobei das Verfahren weiterhin die Erhöhung der Deferoxamin- bzw. HMW-DFO-Dosis umfaßt, um die Wirksamkeit des Antikörpers als Wachstumsinhibitor zu erhöhen.

8. Verwendung nach einem der Ansprüche 6 und 7, wobei in dem Verfahren der Transferrinrezeptor heruntermoduliert und abgebaut wird, dadurch daß die Tumorzellen dem monoklonalen IgG-ATRA ausgesetzt werden.

9. Verwendung nach einem der Ansprüche 6 bis 8, wobei es sich bei den Tumorzellen um hämatopoetische Tumorzellen handelt.

10. Verwendung nach einem der Ansprüche 6 bis 8, wobei es sich bei den Tumorzellen um nichthämatopoetische Tumorzellen handelt.

11. Verwendung von entweder (a) Deferoxamin bzw. chemisch an Hydroxyethylstärke gekoppeltem Deferoxamin (HMW-DFO) oder von (b) Transferrin-Gallium und eines monoklonalen IgG-Antitransferrinrezeptor-Antikörpers zur Herstellung eines Arzneimittels bzw. von Arzneimitteln für die Verwendung in einem *in-vivo*-Verfahren zur Inhibierung von Tumorwachstum, wobei das Verfahren umfaßt, intrazelluläre Eisenspiegel von Tumorzellen zur Erhöhung der Expression des zellulären Transferrinrezeptors in den Tumorzellen zu verringern, indem die Tumorzellen Deferoxamin bzw. HMW-DFO oder Transferrin-Gallium ausgesetzt werden, und anschließend die Tumorzellen einem monoklonalen IgG-Antitransferrinrezeptor-Antikörper (ATRA) auszusetzen.

12. Verwendung nach Anspruch 11, wobei das Verfahren weiterhin die Erhöhung der Deferoxamin- bzw. HMW-DFO-Dosis umfaßt, um die Wirksamkeit des Antikörpers als Wachstumsinhibitor zu erhöhen.

13. Verwendung nach einem der Ansprüche 11 und 12, wobei in dem Verfahren der Transferrinrezeptor heruntermoduliert und abgebaut wird, dadurch daß die Tumorzellen dem monoklonalen IgG-ATRA ausgesetzt werden.

14. Verwendung nach einem der Ansprüche 11 bis 13, wobei es sich bei den Tumorzellen um hämatopoetische Tumorzellen handelt.

15. Verwendung nach einem der Ansprüche 11 bis 13, wobei es sich bei den Tumorzellen um nichthämatopoetische Tumorzellen handelt.

16. Verwendung von Deferoxamin bzw. chemisch an Hydroxyethylstärke gekoppeltem Deferoxamin (HMW-DFO) zur Herstellung eines Arzneimittels für die Verwendung in einem *in-vivo*-Verfahren zur Inhibierung des Einbaus von Thymidin in Tumorzellen, wobei das Verfahren umfaßt, intrazelluläre Eisenspiegel von Tumorzellen zur Inhibierung des Thymidineinbaus zu verringern, indem die Tumorzellen Deferoxamin bzw. HMW-DFO ausgesetzt werden, und anschließend die Tumorzellen einem monoklonalen IgG-Antitransferrinrezeptor-Antikörper auszusetzen und den Inhibitionseffekt zu verstärken.

17. Verwendung eines monoklonalen IgG-Antitransferrinrezeptor-Antikörpers zur Herstellung eines Arzneimittels für die Verwendung in einem *in*-*vivo*-Verfahren zur Inhibierung des Einbaus von Thymidin in Tumorzellen, wobei das Verfahren umfaßt, intrazelluläre Eisenspiegel von Tumorzellen zur Inhibierung des Thymidineinbaus zu verringern, indem die Tumorzellen Deferoxamin bzw. chemisch an Hydroxyethylstärke gekoppeltem Deferoxamin (HMW-DFO) ausgesetzt werden, und anschließend die Tumorzellen einem monoklonalen IgG-Antitransferrinrezeptor-Antikörper auszusetzen und den Inhibitionseffekt zu verstärken.

18. Verwendung von Deferoxamin bzw. chemisch an Hydroxyethylstärke gekoppeltem Deferoxamin (HMW-DFO) und eines monoklonalen IgG-Antitransferrinrezeptor-Antikörpers zur Herstellung eines Arzneimittels bzw. von Arzneimitteln für die Verwendung in einem *in-vivo*-Verfahren zur Inhibierung des Einbaus von Thymidin in Tumorzellen, wobei das Verfahren umfaßt, intrazelluläre Eisenspiegel von Tumorzellen zur Inhibierung des Thymidineinbaus zu verringern, indem die Tumorzellen Deferoxamin bzw. HMW-DFO ausgesetzt werden, und anschließend die Tumorzellen dem monoklonalen IgG-Antitransferrinrezeptor-Antikörper auszusetzen und den Inhibitionseffekt zu verstärken.

19. Verwendung eines Deferoxaminderivats, bei dem es sich um chemisch an Hydroxyethylstärke gekoppeltes Deferoxamin (HMW-DFO) handelt, zur Herstellung eines Arzneimittels für die Verwendung in einem *in*-*vivo*-Verfahren zur Eliminierung von Tumorwachstum, wobei das Verfahren umfaßt, intrazelluläre Eisenspiegel von Tumorzellen zur Erhöhung der Expression des zellulären Transferrinrezeptors in den Tumorzellen zu verringern, indem die Tumorzellen HMW-DFO ausgesetzt werden, und anschließend die Tumorzellen einem monoklonalen IgG-Antitransferrinrezeptor-Antikörper auszusetzen.

20. Verwendung eines monoklonalen IgG-Antitransferrinrezeptor-Antikörpers zur Herstellung eines Arzneimittels für die Verwendung in einem *in-vivo*-Verfahren zur Eliminierung von Tumorwachstum, wobei das Verfahren umfaßt, intrazelluläre Eisenspiegel von Tumorzellen zur Erhöhung der Expression des zellulären Transferrinrezeptors in den Tumorzellen zu verringern, indem die Tumorzellen einem Deferoxaminderivat, bei dem es sich um chemisch an Hydroxyethylstärke gekoppeltes Deferoxamin (HMW-DFO) handelt, ausgesetzt werden, und anschließend die Tumorzellen dem monoklonalen IgG-Antitransferrinrezeptor-Antikörper auszusetzen.

21. Verwendung eines Deferoxaminderivats, bei dem es sich um chemisch an Hydroxyethylstärke gekoppeltes Deferoxamin (HMW-DFO) handelt, und eines monoklonalen IgG-Antitransferrinrezeptor-Antikörpers zur Herstellung eines Arzneimittels für die Verwendung in einem *in*-*vivo*-Verfahren zur Eliminierung von Tumorwachstum, wobei das Verfahren umfaßt, intrazelluläre Eisenspiegel von Tumorzellen zur Erhöhung der Expression des zellulären Transferrinrezeptors in den Tumorzellen zu verringern, indem die Tumorzellen HMW-DFO ausgesetzt werden, und anschließend die Tumorzellen dem monoklonalen IgG-Antitransferrinrezeptor-Antikörper auszusetzen.

## Revendications

1. Utilisation soit (a) de déféroxamine ou de déféroxamine couplée chimiquement à de l'amidon d'hydroxyéthyl (HMW-DFO) soit (b) de transferrine-gallium pour la préparation d'un médicament destiné à un usage dans un procédé *in vivo* d'inhibition de la croissance tumorale, ledit procédé comprenant la déplétion des concentrations en fer intracellulaires de cellules tumorales pour augmenter l'expression d'un récepteur de transferrine cellulaire dans les cellules tumorales en exposant les cellules tumorales à la déféroxamine ou à la HMW-DFO ou à la transferrine-gallium, et en exposant ensuite les cellules tumorales à un anticorps de récepteur d'anti-transferrine (ATRA) IgG monoclonal.

2. Utilisation selon la revendication 1, dans laquelle ledit procédé comprend l'augmentation de la dose de déféroxamine ou de HMW-DFO pour augmenter l'efficacité de l'anticorps comme inhibiteur de croissance.

3. Utilisation selon l'une quelconque des revendications précédentes, dans laquelle, dans ledit procédé, l'exposition des cellules tumorales à l'ATRA IgG monoclonal provoque une baisse de la modulation et une dégradation du récepteur de transferrine.

4. Utilisation selon l'une quelconque des revendications précédentes, dans laquelle les cellules tumorales sont des cellules tumorales hématopoïétiques.

5. Utilisation selon l'une quelconque des revendications 1 à 3, dans laquelle les cellules tumorales sont des cellules tumorales non hématopoïétiques.

6. Utilisation d'un anticorps de récepteur d'anti-transferrine (ATRA) IgG monoclonal pour la préparation d'un médicament destiné à un usage dans un procédé *in vivo* d'inhibition de la croissance tumorale, ledit procédé comprenant la déplétion des concentrations en fer intracellulaires de cellules tumorales pour augmenter l'expression du récepteur de transferrine cellulaire dans les cellules tumorales en exposant les cellules soit (a) à la déféroxamine ou à la déféroxamine couplée chimiquement à de l'amidon d'hydroxyéthyl (HMW-DFO) soit (b) à la transferrine-gallium, et en exposant ensuite les cellules audit anticorps de récepteur d'anti-transferrine IgG monoclonal.

7. Utilisation selon la revendication 6, dans laquelle ledit procédé comprend en outre l'augmentation de la dose de déféroxamine ou de HMW-DFO pour augmenter l'efficacité de l'anticorps comme inhibiteur de croissance.

8. Utilisation selon l'une quelconque des revendications 6 et 7, dans laquelle, dans ledit procédé, l'exposition des cellules tumorales à l'ATRA IgG monoclonal provoque une baisse de la modulation et une dégradation du récepteur de transferrine.

9. Utilisation selon l'une quelconque des revendications 6 à 8, dans laquelle les cellules tumorales sont des cellules tumorales hématopoïétiques.

10. Utilisation selon l'une quelconque des revendications 6 à 8, dans laquelle les cellules tumorales sont des cellules tumorales non hématopoïétiques.

11. Utilisation soit (a) de déféroxamine ou de déféroxamine couplée chimiquement à de l'amidon d'hydroxyéthyl (HMW-DFO) soit (b) de transferrine-gallium, et d'un anticorps de récepteur d'anti-transferrine IgG monoclonal pour la préparation d'un ou plusieurs médicaments destiné(s) à un usage dans un procédé *in vivo* d'inhibition de la croissance tumorale, ledit procédé comprenant la déplétion des concentrations en fer intracellulaires de cellules tumorales pour augmenter l'expression d'un récepteur de transferrine cellulaire dans les cellules tumorales en exposant les cellules tumorales à la déféroxamine ou à la HMW-DFO ou à la transferrine-gallium, et en exposant ensuite les cellules tumorales à l'anticorps de récepteur d'anti-transferrine (ATRA) IgG monoclonal.

12. Utilisation selon la revendication 11, dans laquelle ledit procédé comprend en outre l'augmentation de la dose de déféroxamine ou de HMW-DFO pour augmenter l'efficacité de l'anticorps comme inhibiteur de croissance.

13. Utilisation selon l'une quelconque des revendications 11 et 12, dans laquelle, dans ledit procédé, l'exposition des cellules tumorales à l'ATRA IgG monoclonal provoque une baisse de la modulation et une dégradation du récepteur de transferrine.

14. Utilisation selon l'une quelconque des revendications 11 à 13, dans laquelle les cellules tumorales sont des cellules tumorales hématopoïétiques.

15. Utilisation selon l'une quelconque des revendications 11 à 13, dans laquelle les cellules tumorales sont des cellules tumorales non hématopoïétiques.

16. Utilisation de déféroxamine ou de déféroxamine couplée chimiquement à de l'amidon d'hydroxyéthyl (HMW-DFO) pour la préparation d'un médicament destiné à un usage dans un procédé *in vivo* d'inhibition de l'incorporation de thymidine à des cellules tumorales, ledit procédé comprenant la déplétion des concentrations en fer intracellulaires de cellules tumorales pour inhiber l'incorporation de thymidine en exposant les cellules tumorales à la déféroxamine ou à la HMW-DFO et en exposant les cellules tumorales à un anticorps de récepteur d'anti-transferrine IgG monoclonal et en améliorant l'effet inhibiteur.

17. Utilisation d'un anticorps de récepteur d'anti-transferrine IgG monoclonal pour la préparation d'un médicament destiné à un usage dans un procédé *in vivo* d'inhibition de l'incorporation de thymidine à des cellules tumorales, ledit procédé comprenant la déplétion des concentrations en fer intracellulaires des cellules tumorales pour inhiber l'incorporation de thymidine en exposant les cellules tumorales à la déféroxamine ou à la déféroxamine couplée chimiquement à de l'amidon d'hydroxyéthyl (HMW-DFO) et en exposant les cellules tumorales à un anticorps de récepteur d'anti-transferrine IgG monoclonal et en améliorant l'effet inhibiteur.

18. Utilisation de déféroxamine ou de déféroxamine couplée chimiquement à de l'amidon d'hydroxyéthyl (HMW-DFO) et d'un anticorps de récepteur d'anti-transferrine IgG monoclonal pour la préparation d'un ou plusieurs médicaments destiné(s) à un usage dans un procédé *in vivo* d'inhibition de l'incorporation de thymidine à des cellules tumorales, ledit procédé comprenant la déplétion des concentrations en fer intracellulaires des cellules tumorales pour inhiber l'incorporation de thymidine en exposant les cellules tumorales à la déféroxamine ou à la HMW-DFO et en exposant les cellules tumorales à l'anticorps de récepteur d'anti-transferrine IgG monoclonal et en améliorant l'effet inhibiteur.

19. Utilisation d'un dérivé de déféroxamine qui est une déféroxamine couplée chimiquement à de l'amidon d'hydroxyéthyl (HMW-DFO) pour la préparation d'un médicament destiné à un usage dans un procédé *in vivo* d'élimination de la croissance tumorale, ledit procédé comprenant la déplétion des concentrations en fer intracellulaires de cellules tumorales pour augmenter l'expression d'un récepteur de transferrine cellulaire dans les cellules tumorales en exposant les cellules tumorales à la HMW-DFO et en exposant les cellules tumorales à un anticorps de récepteur d'anti-transferrine IgG monoclonal.

20. Utilisation d'un anticorps de récepteur d'anti-transferrine IgG monoclonal pour la préparation d'un médicament destiné à un usage dans un procédé *in vivo* d'élimination de la croissance tumorale, ledit procédé comprenant la déplétion des concentrations en fer intracellulaires de cellules tumorales pour augmenter l'expression d'un récepteur de transferrine cellulaire dans les cellules tumorales en exposant les cellules tumorales à un dérivé de déféroxamine qui est une déféroxamine couplée chimiquement à de l'amidon d'hydroxyéthyl (HMW-DFO) et en exposant les cellules tumorales à l'anticorps de récepteur d'anti-transferrine IgG monoclonal.

21. Utilisation d'un dérivé de déféroxamine qui est une déféroxamine couplée chimiquement à de l'amidon d'hydroxyéthyl (HMW-DFO) et d'un anticorps de récepteur d'anti-transferrine IgG monoclonal pour la préparation d'un médicament destiné à un usage dans un procédé *in vivo* d'élimination de la croissance tumorale, ledit procédé comprenant la déplétion des concentrations en fer intracellulaires de cellules tumorales pour augmenter l'expression d'un récepteur de transferrine cellulaire dans les cellules tumorales en exposant les cellules tumorales à la HMW-DFO et en exposant les cellules tumorales à l'anticorps de récepteur d'anti-transferrine IgG monoclonal.
